# EUROPEAN PATENT SPECIFICATION

(11) **EP 1 931 372 B1**
(45) Date of publication and mention of the grant of the patent: **29.04.2015**
(21) Application number: 06779285.3
(22) Date of filing: 05.09.2006
(51) Int. Cl.: A61P 21/04, A61K 38/17

(54) **METHOD OF TREATING MYASTHENIA GRAVIS**
VERFAHREN ZUR BEHANDLUNG VON MYASTHENIA GRAVIS
PROCÉDÉ DE TRAITEMENT DE LA MYASTHÉNIE GRAVE

(30) Priority: 09.09.2005 GB 0518443
(43) Date of publication of application: 18.06.2008
(73) Proprietor: Volution Immuno Pharmaceuticals SA, 3461-1211 Geneve 3 (CH)
(72) Inventor: HAMER, John, Reading Berkshire RG2 6UG (GB); WESTON-DAVIES, Wynne, Reading Berkshire RG2 6UG (GB)
(74) Representative: Goodfellow, Hugh Robin
(86) International application number: PCT/GB2006/003265
(87) International publication number: WO 2007/028968

(56) References cited:
- WO-A-2005/079363
- WO-A2-2004/106369
- WO-A2-2005/023866
- NUNN M A ET AL: "Complement inhibitor of C5 activation from the soft tick Ornithodoros moubata" JOURNAL OF IMMUNOLOGY 15 FEB 2005 UNITED STATES, vol. 174, no. 4, 15 February 2005 (2005-02-15), pages 2084-2091, XP002407335 ISSN: 0022-1767
- SAHU ARVIND ET AL: "Complement inhibitors: A resurgent concept in anti-inflammatory therapeutics" IMMUNOPHARMACOLOGY, vol. 49, no. 1-2, August 2000 (2000-08), pages 133-148, XP002407384 ISSN: 0162-3109
- RICHMAN DAVID P ET AL: "Antibody effector mechanisms in myasthenia gravis: The complement hypothesis" ANNALS OF THE NEW YORK ACADEMY OF SCIENCES; MYASTHENIA GRAVIS AND RELATED DISEASES: DISORDERS OF THE NEUROMUSCULAR JUNCTION NEW YORK ACADEMY OF SCIENCES {A}, 2 EAST 63RD STREET, NEW YORK, NEW YORK 10021, USA SERIES : ANNALS OF THE NEW YORK ACADEMY OF, 1998, pages 450-465, XP002407336 & NINTH INTERNATIONAL CONFERENCE; SANTA MONICA, CALIFORNIA, USA; MAY 7-10, 1997 ISSN: 1-57331-119-7 1-57331-120-0
- SOLTYS ET. AL: "Novel Complement Inhibitor Limits Severity of Experimental Myasthenia gravis", ANNALS OF NEUROLOGY (IN PRESS), pages 1-19,

## Description

The present invention relates to the use of agents that bind the complement protein C5 in the treatment of diseases associated with inappropriate complement activation, and in particular in the treatment of myasthenia gravis.

### Background to the invention

The complement system is an essential part of the body's natural defence mechanism against foreign invasion and is also involved in the inflammatory process. More than 30 proteins in serum and at the cell surface are involved in complement system function and regulation. Recently it has become apparent that, as well as the ~35 known components of the complement system which may be associated with both beneficial and pathological processes, the complement system itself interacts with at least 85 biological pathways with functions as diverse as angiogenesis, platelet activation, glucose metabolism and spermatogenesis (Mastellos, D., et al., Clin Immunol, 2005. 115(3): p. 225-35).

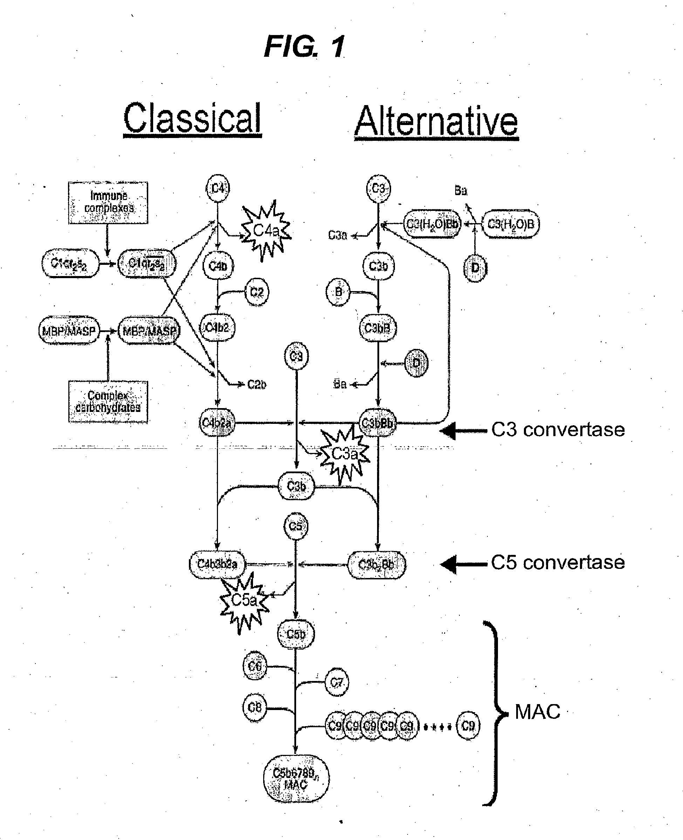

The complement system is activated by the presence of foreign antigens. Three activation pathways exist: (1) the classical pathway which is activated by IgM and IgG complexes or by recognition of carbohydrates; (2) the alternative pathway which is activated by non-self surfaces (lacking specific regulatory molecules) and by bacterial endotoxins; and (3) the lectin pathway which is activated by binding of manna-binding lectin (MBL) to mannose residues on the surface of a pathogen. The three pathways comprise parallel cascades of events that result in the production of complement activation through the formation of similar C3 and C5 convertases on cell surfaces resulting in the release of acute mediators of inflammation (C3a and C5a) and formation of the membrane attack complex (MAC). The parallel cascades involved in the classical and alternative pathways are shown in Figure 1.

Complement can be activated inappropriately under certain circumstances leading to undesirable local tissue destruction. Inappropriate complement activation has been shown to play a role in a wide variety of diseases and disorders including acute pancreatitis, Alzheimer's disease, allergic encephalomyelitis, allotransplatation, asthma, adult respiratory distress syndrome, burn injuries, Crohn's disease, glomerulonephritis, haemolytic anaemia, haemodialysis, hereditary angioedema, ischaemia reperfusion injuries, multiple system organ failure, multiple sclerosis, myasthenia gravis, ischemic stroke, myocardial infarction, psoriasis, rheumatoid arthritis, septic shock, systemic lupus erythematosus, stroke, vascular leak syndrome, transplantation rejection and inappropriate immune response in cardiopulmonary bypass operations. Inappropriate activation of the complement system has thus been a target for therapeutic intervention for many years and numerous complement inhibitors targeting different parts of the complement cascade are under development for therapeutic use.

In ischemic stroke and myocardial infarction, the body recognises the dead tissue in the brain or heart as foreign and activates complement so causing further local damage. Similarly in cardiopulmonary bypass operations, the body recognises the plastic surfaces in the machine as foreign, activates complement and can result in vascular damage. In autoimmune diseases, the body may wrongly recognise itself as foreign and activate complement with local tissue damage (*e.g.* joint destruction in rheumatoid arthritis and muscle weakness in myasthenia gravis).

Myasthenia gravis is a chronic autoimmune disease that results in progressive fatigue, loss of muscle tone and increasing paralysis. These symptoms are caused by inappropriate activation of complement resulting in an immune response directed against the nicotinic acetylcholine receptor (AchR) which leads, in turn, to reduced neuromuscular transmission. Myasthenia gravis may occur in association with other diseases such as a thymic tumor or thyrotoxicosis, as well as with rheumatoid arthritis and lupus erythematosus.

There is currently no cure for myasthenia gravis. The disease is usually treated initially using anticholinesterase agents, such as neostigmine bromide (Prostigmin) and pyridostigmine bromide (Mestinon), which help improve neuromuscular transmission and increase muscle strength. Treatment with anticholinesterase agents is, however, associated with adverse side effects caused from acetylcholine accumulation including gastrointestinal complaints and increased bronchial and oral secretions. In addition, although anticholinesterase agents often provide symptomatic benefit, they do not influence the course of the disease. Patients who do not respond to anticholinerterase agents may also be treated with long-term immunosuppressive drugs such as the cortocosteroid prednisone, or other immunosuppressant drugs such as cyclosporine, azathioprine and cyclophosphamide. These immunosuppressant drugs are, however, associated with serious side effect. Corticosteroids side effects include weight gain, osteoporosis, hypertension and glaucoma. Azathioprine and cyclosporine are associated with liver dysfunction and an increased risk of malignancy. In some cases, thymectomy is recommended as an alternative to drugs but the response is unpredictable and symptoms of the disease may continue for months or years after surgery.

Experimental autoimmune myasthenia gravis (EAMG) may be induced in animal models by immunisation with purified AChR or anti-AChR antibodies and these models are useful in assessing the effect of complement inhibitors on the progression of the disease. The complement inhibitor soluble complement receptor 1 (sCRI) has been shown to delay weight loss and reduce clinical signs of EAMG, suggesting that this molecule may be useful in the treatment for myasthenia gravis (Piddlesden et al, J. Neuroimmunol., 1996, 71: 173-177). However, daily injections of sCR1 were required to achieve these effects and, although sCR1 reduced weight loss, it did not completely prevent it. sCR1 does not therefore completely prevent the symptoms of myasthenia gravis.

Furthermore, sCR1 acts by binding early products of the complement cascade, C3b and C4b. The complement system plays an important and valuable role in defence against pathogens and many of the early by-products of the cascade are important in the recognition and opsonisation of pathogenic organisms. For this reason, therapeutic intervention in the earlier stages of the classical and alternative pathways is considered to carry the risk of increased susceptibility to microbial infection (Roos, A., et al., Immunobiology, 2002, 205(4-5): p. 595-609).

There is thus a great need for agents that improve upon the currently available treatments for myasthenia gravis.

### Summary of the invention:

Accordingly, the invention provides an agent derived from a haematophagous arthropod that binds complement C5 for use in the treatment or prevention of myasthenia gravis, wherein the agent that binds complement C5 is defined in claim 1.

The invention also provides the use of a therapeutically or prophylactically effective amount of an agent derived from a haematophagous arthropod that binds complement C5 in the manufacture of a medicament for treating or preventing myasthenia gravis, wherein the agent that binds complement C5 is defined in claim 17.

Preferably, the agent acts to prevent the cleavage of complement C5 by C5 convertase into complement C5a and complement C5b-9.

The complement C5 protein, also referred to herein as C5, is cleaved by the C5 convertase enzyme, itself formed from C3a, an earlier product of the alternative pathway (Figure 1). The products of this cleavage include an anaphylatoxin C5a and a lytic complex C5b - 9 also known as membrane attack complex (MAC). C5a is a highly reactive peptide implicated in many pathological inflammatory processes including neutrophil and eosinophil chemotaxis, neutrophil activation, increased capillary permeability and inhibition of neutrophil apoptosis (Guo, R.F. and P.A. Ward, Annu Rev Immunol, 2005, 23: p. 821-52).

MAC is associated with other important pathological processes including rheumatoid arthritis (Neumann, E., et al., Arthritis Rheum, 2002. 46(4): p. 934-45; Williams, A.S., et al., Arthritis Rheum, 2004, 50(9): p. 3035-44), proliferative glomerulonephritis (Quigg, R.J., Curr Dir Autoimmun, 2004. 7: p. 165-80, idiopathic membranous nephropathy (Papagianni, A.A., et al., Nephrol Dial Transplant, 2002, 17(1): p. 57-63), proteinurea (He, C., et al., J Immunol, 2005. 174(9): p. 5750-7), demyelination after acute axonal injury (Mead, R.J., et al., J Immunol, 2002. 168(1): p. 458-65) and is also responsible for acute graft rejection following xenotransplantation (Nakashima, S., et al., J Immunol, 2002. 169(8): p. 4620-7).

C5a has become a target of particular interest in the field of complement-associated disorders (Mizuno, M. and D.S. Cole, Expert Opin Investig Drugs, 2005. 14(7): p. 807-21). Although C5a has many well-recognised pathological associations, the effects of its depletion in humans appear to be limited. Monoclonal antibodies and small molecules that bind and inhibit C5a or C5a receptors have been developed to treat various autoimmune diseases. These molecules do not, however, prevent the release of MAC.

In contrast, administration of an agent that binds C5 according to the first aspect of the invention, inhibits both the C5a peptide and the MAC. Surprisingly, it has been found that inhibition of both C5a and the MAC completely attenuates clinical symptoms associated with myasthenia gravis. Furthermore, because C5 is a late product of the classical and alternative complement pathways, inhibition of C5 is less likely to be associated with risks of concomitant infection that exist when targeting earlier products in the cascade (Allegretti, M., et al.,. Curr Med Chem, 2005. 12(2): p. 217-36).

The ability of an agent to bind C5 may be determined by standard *in vitro* assays known in the art, for example by western blotting following incubation of the protein on the gel with labelled C5. Preferably, the agent according to the invention binds C5 with an IC₅₀ of less than 0.2 mg/ml, preferably less than 0.1 mg/ml, preferably less than 0.05 mg/ml, preferably less than 0.04 mg/ml, preferably less than 0.03 mg/ml, preferably 0.02 mg/ml, preferably less than 1 µg/ml, preferably less than 100ng/ml, preferably less than 10ng/ml more preferably still, less than 1ng/ml.

The agent that binds C5 is derived from a haematophagous arthropod. The term "haematophagous arthropod" includes all arthropods that take a blood meal from a suitable host, such as insects, ticks, lice, fleas and mites. Preferably, the agent is derived from a tick, preferably from the tick *Ornithodoros moubata.*

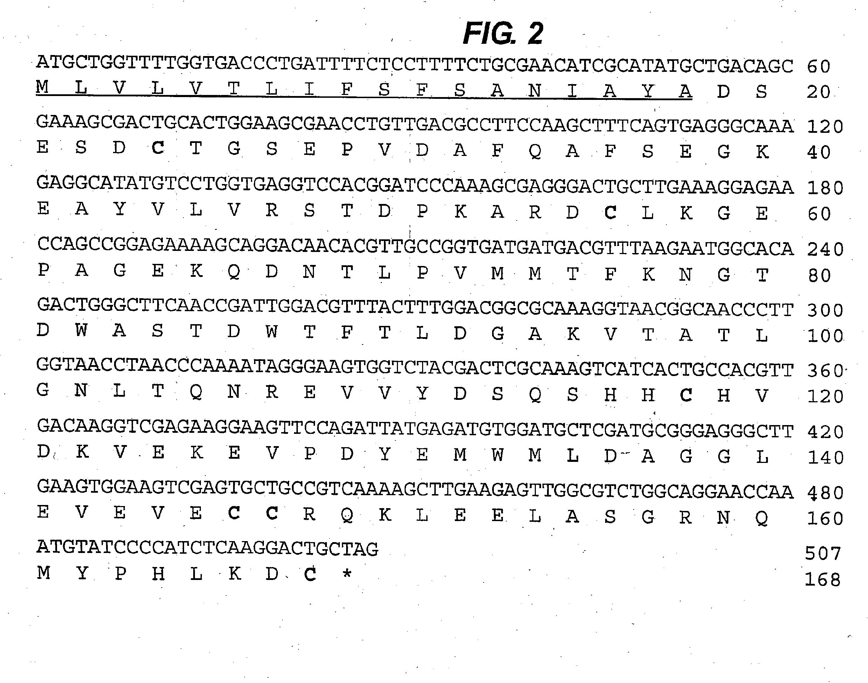

According to the invention, the agent that binds C5 is a protein comprising amino acids 19 to 168 of the amino acid sequence in Figure 2 or is a functional equivalent of this protein as defined in claim 1 or claim 17. The agent that binds C5 may be a protein consisting of amino acids 19 to 168 of the amino acid sequence in Figure 2 or be a functional equivalent of this protein.

According to the invention, the protein may comprise or consist of amino acids 1 to 168 of the amino acid sequence in Figure 2, or be a functional equivalent thereof. The first 18 amino acids of the protein sequence given in Figure 2 form a signal sequence which is not required for C5 binding activity and so this may optionally be dispensed with, for example, for efficiency of recombinant protein production.

The protein having the amino acid sequence given in Figure 2, also referred to herein as the EV576 protein, was isolated from the salivary glands of the tick *Ornithodoros moubata.* EV576 is an outlying member of the lipocalin family and is the first lipocalin family member shown to inhibit complement activation. The EV576 protein inhibits the alternative, classical and lectin complement pathways by binding C5 and preventing its cleavage by C5 convertase into Complement C5a and Complement C5b - 9, thus inhibiting both the action of C5a peptide and the MAC. The term "EV576 protein", as used herein, refers to the sequence given in Figure 2 with or without the signal sequence.

The EV576 protein and the ability of this protein to inhibit complement activation has been disclosed in WO2004/106369, where the EV576 protein was referred to as the "OmCI protein". It has now been found that the EV576 protein is surprisingly effective in the treatment and prevention of myasthenia gravis. The data presented herein demonstrate that a single injection of EV576 totally attenuates weight loss and muscular weakness in the EAMG in mice for at least 7 days. EV576 is thus more effective in the treatment and prevention of EAMG than sCR1 which, as discussed above, only reduced the clinical symptoms of myasthenia gravis when administered on a daily basis (Piddlesden et al, J. Neuroimmunol., 1996, 71: 173-177). The surprising effectiveness of EV576 in the treatment of myasthenia gravis appears to be due to the fact that it acts by binding C5, thus inhibiting the activity of C5a and MAC. In addition, the data presented herein demonstrate that rEV576 is effective in models of both early mild myasthenia gravis and severe late stage myasthenic crises.

According to a further embodiment of the invention, the agent may be a nucleic acid molecule encoding the EV576 protein or a functional equivalent thereof. For example, gene therapy may be employed to effect the endogenous production of the EV576 protein by the relevant cells in the subject, either *in vivo* or *ex vivo*. Another approach is the administration of "naked DNA" in which the therapeutic gene is directly injected into the bloodstream or into muscle tissue.

Preferably, such a nucleic acid molecule comprises or consists of bases 53 to 507 of the nucleotide sequence in Figure 2. This nucleotide sequence encodes the EV576 protein in Figure 2 without the signal sequence. The first 54 bases of the nucleotide sequence in Figure 2 encode the signal sequence of which is not required for complement inhibitory activity. Alternatively, the nucleic acid molecule may comprise or consist of bases 1 to 507 of the nucleic acid sequence in Figure 2, which encodes the protein with the signal sequence.

The EV576 protein has been demonstrated to bind to C5 and prevent its cleavage by C5 convertase in rat, mouse and human serum with an IC₅₀ of approximately 0.02mg/ml. Preferably, functional equivalents of the EV576 protein which retain the ability to bind C5 with an IC₅₀ of less than 0.2 mg/ml, preferably less than 0.1 mg/ml, preferably less than 0.05 mg/ml, preferably less than 0.02 mg/ml, preferably less than 1 µg/ml, preferably less than 100ng/ml, preferably less than 10ng/ml, more preferably still, less than 1ng/ml.

The serum beta half-life of ¹²⁵I labelled EV576 in rats has been found to be 30 - 38 hours. Preferably, the EV576 protein and its functional equivalents retain a half-life of greater than 20 hours, preferably greater than 25 hours, preferably greater than 30 hours, preferably greater than 40 hours, preferably greater than 50 hours, preferably greater than 100 hours.

In one respect, the term "functional equivalent" is used herein to describe homologues and fragments of the EV576 protein which retain its ability to bind C5, and to prevent the cleavage of complement C5 by C5 convertase into complement C5a and complement C5b-9. The term "functional equivalent" also refers to molecules that are structurally similar to the EV576 protein or that contain similar or identical tertiary structure, particularly in the environment of the active site or active sites of the EV576 protein that binds to C5, such as synthetic molecules.

The term "homologue" is meant to include reference to paralogues and orthologues of the EV576 sequence that is explicitly identified in Figure 2, including, for example, the EV576 protein sequence from other tick species, including *Rhipicephalus appendiculatus, R. sanguineus, R. bursa, A. americanum*, *A. cajennense, A. hebraeum, Boophilus microplus, B. annulatus, B. decoloratus, Dermacentor reticulatus, D. andersoni*, *D. marginatus, D. variabilis, Haemaphysalis inermis, Ha. leachii, Ha. punctata, Hyalomma anatolicum anatolicum, Hy. dromedarii, Hy. marginatum marginatum, Ixodes ricinus, I. persulcatus, I. scapularis, I. hexagonus, Argas persicus, A. reflexus, Ornithodoros erraticus, O. moubata moubata, O. m. porcinus, and O, savignyi.* The term "homologue" is also meant to include the equivalent EV576 protein sequence from mosquito species, including those of the *Culex, Anopheles* and *Aedes* genera, particularly *Culex quinquefasciatus, Aedes aegypti* and *Anopheles gambiae;* flea species, such as *Ctenocephalides felis* (the cat flea); horseflies; sandflies; blackflies; tsetse flies; lice; mites; leeches; and flatworms. The native EV576 protein is thought to exist in *O*. *moubata* in another three forms of around 18kDa and the term "homologue" is meant to include these alternative forms of EV576.

Methods for the identification of homologues of the EV576 sequence given in Figure 2 will be clear to those of skill in the art. For example, homologues may be identified by homology searching of sequence databases, both public and private. Conveniently, publicly available databases may be used, although private or commercially-available databases will be equally useful, particularly if they contain data not represented in the public databases. Primary databases are the sites of primary nucleotide or amino acid sequence data deposit and may be publicly or commercially available. Examples of publicly-available primary databases include the GenBank database (http://www.ncbi.nlm.nih.gov/), the EMBL database (http://www.ebi.ac.uk/), the DDBJ database (http://www.ddbj.nig.ac.jp/), the SWISS-PROT protein database (http://expasy.hcuge.ch/), PIR (http://pir.georgetown.edu/), TrEMBL (http://www.ebi.ac.uk/), the TIGR databases (see http://www.tigr.org/tdb/index.html), the NRL-3D database (http://www.nbrfa.georgetown.edu), the Protein Data Base (http://www.rcsb.org/pdb), the NRDB database (ftp://ncbi.nlm.nih.gov/pub/nrdb/README), the OWL database (http://www.biochem.ucl.ac.uk/bsm/dbbrowser/OWL/) and the secondary databases PROSITE (http://expasy.hcuge.ch/sprot/prosite.html), PRINTS (http://iupab.leeds.ac.uk/bmb5dp/prints.html), Profiles (http://ulrec3.unil.ch/software/PFSCAN_form.html), Pfam (http://www.sanger.ac.uk/software/pfam), Identify (http://dna.stanford.edu/identify/) and Blocks (http://www.blocks.fhcrc.org) databases. Examples of commercially-available databases or private databases include PathoGenome (Genome Therapeutics Inc.) and PathoSeq (Incyte Pharmaceuticals Inc.).

Typically, greater than 30% identity between two polypeptides (preferably, over a specified region such as the active site) is considered to be an indication of functional equivalence and thus an indication that two proteins are homologous. Preferably, proteins that are homologues have a degree of sequence identity with the EV576 protein sequence identified in Figure 2 of greater than 60%. More preferred homologues have degrees of identity of greater than 70%, 80%, 90%, 95%, 98% or 99%, respectively with the EV576 protein sequence given in Figure 2. Percentage identity, as referred to herein, is as determined using BLAST version 2.1.3 using the default parameters specified by the NCBI (the National Center for Biotechnology Information; http://www.ncbi.nlm.nih.gov/) [Blosum 62 matrix; gap open penalty=11 and gap extension penalty=1].

Homologues of the EV576 protein sequence given in Figure 2 include mutants containing amino acid substitutions, insertions or deletions from the wild type sequence, for example, of 1, 2, 3, 4, 5, 7, 10 or more amino acids, provided that such mutants retain the ability to bind C5. Mutants thus include proteins containing conservative amino acid substitutions that do not affect the function or activity of the protein in an adverse manner. This term is also intended to include natural biological variants (e.g. allelic variants or geographical variations within the species from which the EV576 proteins are derived). Mutants with improved ability to bind C5 may also be designed through the systematic or directed mutation of specific residues in the protein sequence.

Fragments of the EV576 protein and of homologues of the EV576 protein are also embraced by the term "functional equivalents" providing that such fragments retain the ability to bind C5. Fragments may include, for example, polypeptides derived from the EV576 protein sequence which are less than 150 amino acids, less than 125 amino acids, less than 100 amino acids, less than 75 amino acids, less than 50 amino acids, or even 25 amino acids or less, provided that these fragments retain the ability to bind to complement C5. Included as such fragments are not only fragments of the *O. moubata* EV576 protein that is explicitly identified herein in Figure 2, but also fragments of homologues of this protein, as described above. Such fragments of homologues will typically possess greater than 60% identity with fragments of the EV576 protein sequence in Figure 2, although more preferred fragments of homologues will display degrees of identity of greater than 70%, 80%, 90%, 95%, 98% or 99%, respectively with fragments of the EV576 protein sequence in Figure 2. Fragments with improved may, of course, be rationally designed by the systematic mutation or fragmentation of the wild type sequence followed by appropriate activity assays. Fragments may exhibit similar or greater affinity for C5 as EV576 and may have the same or greater IC₅₀ for C5.

A functional equivalent used according to the invention maybe a fusion protein, obtained, for example, by cloning a polynucleotide encoding the EV576 protein in frame to the coding sequences for a heterologous protein sequence. The term "heterologous", when used herein, is intended to designate any polypeptide other than the EV576 protein or its functional equivalent. Example of heterologous sequences, that can be comprised in the soluble fusion proteins either at N- or at C-terminus, are the following: extracellular domains of membrane-bound protein, immunoglobulin constant regions (Fc region), multimerization domains, domains of extracellular proteins, signal sequences, export sequences, or sequences allowing purification by affinity chromatography. Many of these heterologous sequences are commercially available in expression plasmids since these sequences are commonly included in the fusion proteins in order to provide additional properties without significantly impairing the specific biological activity of the protein fused to them (Terpe K, Appl Microbiol Biotechnol, 60: 523-33, 2003). Examples of such additional properties are a longer lasting half-life in body fluids, the extracellular localization, or an easier purification procedure as allowed by a tag such as a histidine or HA tag.

The EV576 protein and functional equivalents thereof, may be prepared in recombinant form by expression in a host cell. Such expression methods are well known to those of skill in the art and are described in detail by Sambrook *et al* (2000) and Fernandez & Hoeffler (1998). Recombinant forms of the EV576 protein and functional equivalents thereof are preferably unglycosylated.

The proteins and fragments for use in the present invention can also be prepared using conventional techniques of protein chemistry. For example, protein fragments may be prepared by chemical synthesis. Methods for the generation of fusion proteins are standard in the art and will be known to the skilled reader. For example, most general molecular biology, microbiology recombinant DNA technology and immunological techniques can be found in Sambrook *et al.* (2000) or Ausubel *et al.* (1991).

The subject to which the agent that binds C5 is administered is preferably a mammal, preferably a human. The subject to which the agent that binds C5 is administered may also be suffering from a further disease with which myasthenia gravis is associated, such as a thymic tumor, thyrotoxicosis, rheumatoid arthritis and lupus erythematosus.

The agent is administered in a therapeutically or prophylactically effective amount. The term "therapeutically effective amount" refers to the amount of agent needed to treat or ameliorate a targeted disease. The term "prophylactically effective amount" used herein refers to the amount of agent needed to prevent a targeted disease.

Preferably, the dose of the agent is sufficient to bind as much available C5 as possible in the subject, more preferably, all available C5. Preferably, the dose of the agent supplied is at least twice the molar dose needed to bind all available C5 in the subject. The dose of the agent supplied may be 2.5 times, 3 times or 4 times the molar dose needed to bind all available C5 in the subject. In one embodiment, the dose is from 1 mg/kg to 15 mg/kg. Preferably, the dose is from 1 mg/kg (mass of drug compared to mass of patient) to 10 mg/kg, preferably 2 mg/kg to 8 mg/kg.

The frequency with which the dose needs to be administered will depend on the half-life of the agent involved. Where the agent is the EV576 protein or a functional equivalent thereof, the dose may be administered on a daily basis, a twice daily basis, or every two, three, four days, five, six, seven, 10, 15 or 20 days or more.

The exact dosage and the frequency of doses may also be dependent on the patient's status at the time of administration. Factors that may be taken into consideration when determining dosage include the severity of the disease state in the patient, the general health of the patient, the age, weight, gender, diet, time and frequency of administration, drug combinations, reaction sensitivities and the patient's tolerance or response to therapy. The precise amount can be determined by routine experimentation, but may ultimately lie with the judgement of the clinician.

The agent will generally be administered as part of a pharmaceutically acceptable carrier. The term "pharmaceutically acceptable carrier", as used herein, includes gene, polypeptides, antibodies, liposomes, polysaccharides, polylactic acids, polyglycolic acids and inactive virus particles or indeed any other agent provided that the carrier does not itself induce toxicity effects or cause the production of antibodies that are harmful to the individual receiving the pharmaceutical composition. Pharmaceutically acceptable carriers may additionally contain liquids such as water, saline, glycerol, ethanol or auxiliary substances such as wetting or emulsifying agents, pH buffering substances and the like, The pharmaceutical carrier employed will thus vary depending on the route of administration. Carriers may enable the pharmaceutical compositions to be formulated into tablets, pills, dragees, capsules, liquids, gels, syrups, slurries, suspensions to aid intake by the patient. A thorough discussion of pharmaceutically acceptable carriers is available in Remington's Pharmaceutical Sciences (Mack Pub. Co., N.J. 1991).

The agent may be delivered by any known route of administration. The agent may be delivered by a parenteral route (*e.g.* by injection, either subcutaneously, intraperitoneally, intravenously or intramuscularly or delivered to the interstitial space of a tissue). The compositions can also be administered into a lesion. Other modes of administration include oral and pulmomary administration, suppositories, and transdermal or transcutaneous applications, needles, and hyposprays.

The agent that binds C5 may be administered alone or as part of a treatment regimen also involving the administration of other drugs currently used in the treatment of patients with myasthenia gravis. For example, the agent may be administered in combination with anticholinesterase agents, such as neostigmine and pyridostigmine, or immunosuppressive drugs, such as prednisone, cyclosporine, and azathioprine. Combinations of drug treatments may have an additive or synergistic effect on treatment of the disease.

The invention provides an agent derived from a haemotaphagous arthropod that binds C5, wherein the agent is EV576 protein or a functional equivalent thereof, and the agent is administered as part of a treatment regimen involving the administration of an anticholinesterase agent and/or an immunosuppressive drug, for use in the treatment or prevention of myasthenia gravis.

The agent that binds C5 may be administered simultaneously, sequentially or separately with the other drug(s). For example, the agent that binds C5 may be administered before or after administration of the other drug(s).

The invention thus provides the use of an agent that binds C5, wherein the agent is EV576 protein or a functional equivalent thereof, in the manufacture of a medicament for treating myasthenia gravis in a subject, wherein said subject has been pre-treated with an anticholinesterase agent and/or an immunosuppressive drug. The invention also provides the use of an anticholinesterase agent and/or an immunosuppressive drug in the manufacture of a medicament for treating myasthenia gravis in a subject wherein said subject has been pre-treated with an agent that binds C5, wherein the agent is EV576 protein or a functional equivalent thereof.

The agent that binds C5 may also be administered as part of a treatment regimen also involving the administration of other drugs currently used in the treatment of other diseases with which myasthenia gravis is associated, such as a thymic tumor, thyrotoxicosis, rheumatoid arthritis and lupus erythematosus. The agent that binds C5 may be administered simultaneously, sequentially or separately with the other drug(s). For example, the agent that binds C5 may be administered before or after administration of the other drug(s).

Various aspects and embodiments of the present invention will now be described in more detail by way of example. It will be appreciated that modification of detail may be made without departing from the scope of the invention.

### Brief description of Figures:

**Figure 1****:** Schematic diagram of classical and alternative pathways of complement activation. Enzymatic components, dark grey. Anaphylatoxins enclosed in starbursts.
**Figure 2****:** Primary sequence of EV576. Signal sequence underlined. Cysteine residues in bold type. Nucleotide and amino acid number indicated at right.
**Figure 3****:** Purification of EV576 from tick salivary gland extract (SGE). A) Anion exchange chromatography. B) Classical haemolytic assay of fractions. C) Reducing SDS-PAGE. D) RP-HPLC.
**Figure 4****:** Mechanism of action of EV576. A) No effect on C3a production. B) Prevents C5a production. C) Binds directly to C5.
**Figure 5****:** Recombinant EV576. A) Recombinant EV576 (rEV576) inhibits complement as effectively as native EV576. B) Structure of EV576.
**Figure 6****:** Effect of rEV576 in experimental myasthenia gravis model. A) rEV576 prevents weight loss compared with control animals. B) Clinical scores in animals treated with rEV576 compared with control animals. C) Raw data for clinical scores.
**Figure 7****:** Effect of rEV576 in chronic experimental myasthenia gravis model. A) In animals with severe EAMG, rEV576. prevents weight loss and death compared with control animals. B) In animals with severe EAMG, rEV576 treatment gives significant improvement in clinical score and grip strength compared with control animals. C) In animals with mild EAMG, rEV576 prevents weight loss compared with control animals.
**Figure 8****:** Effect of rEV576 in chronic experimental myasthenia gravis model. A) AchR antibodies showed no differences between treated, untreated, mild EAMG or severe EAMG groups B) total complement hemolytic activity of rat serum from rats exhibiting both severe and mild EAMG.
**Figure 9****:** Effect of rEV576 in chronic experimental myasthenia gravis model. A) Cytotoxicity of rat serum from rats exhibiting severe EAMG. B) Cytotoxicity of rat serum from rats exhibiting mild EAMG.

### Examples

### 1. Mechanism of action and inhibitory concentration.

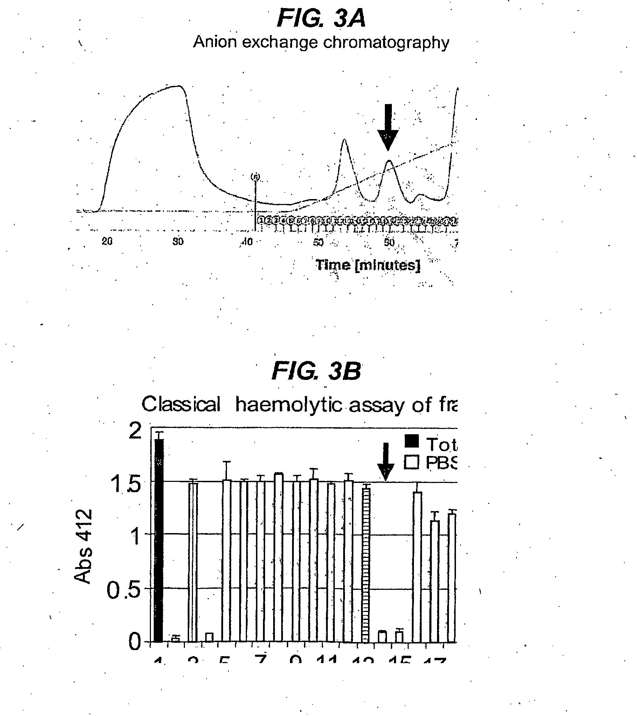

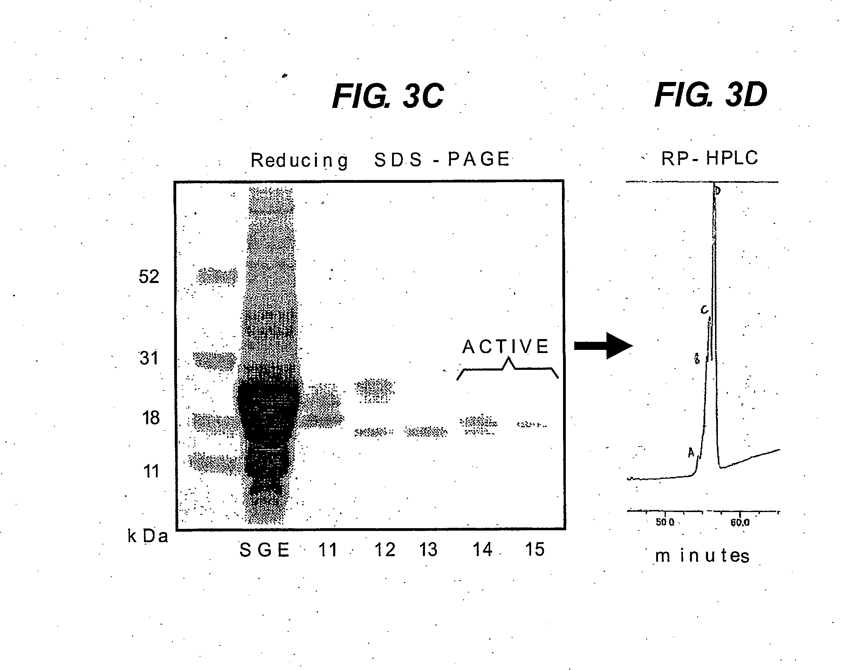

EV576 was purified from salivary gland extracts of the soft tick *Orthinodoros moubata* by SDS-PAGE and RP-HPLC of fractions of salivary gland extract found to contain complement inhibitory activity by classical haemolytic assays (Figure 3) as disclosed in WO2004/106369.

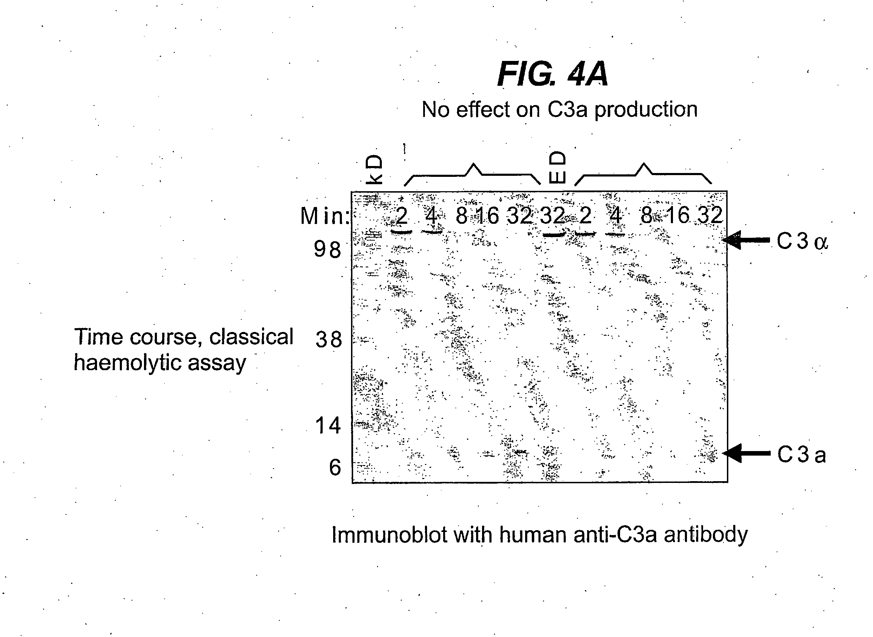

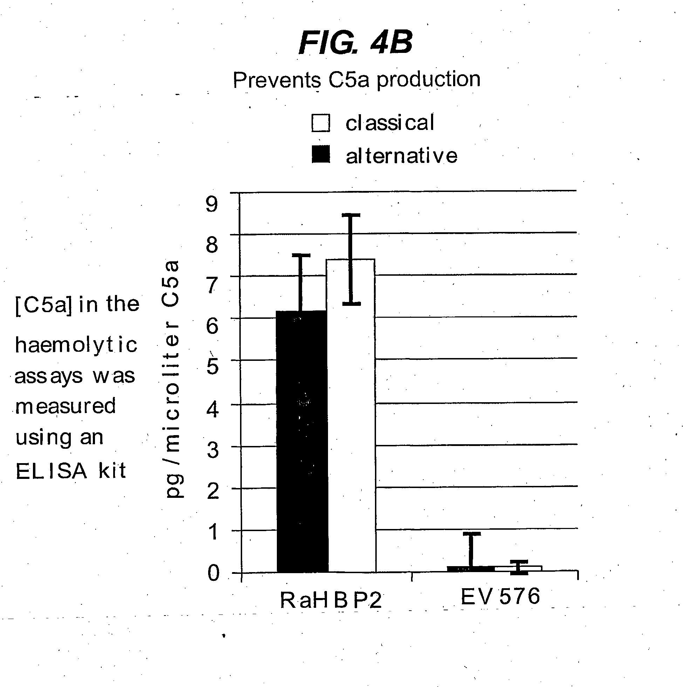

EV576 inhibits both human and guinea pig classical and alternative pathways. It has no effect on the rate of C3a production (Figure 4A) but prevents cleavage of C5a from C5 (Figure 4B).

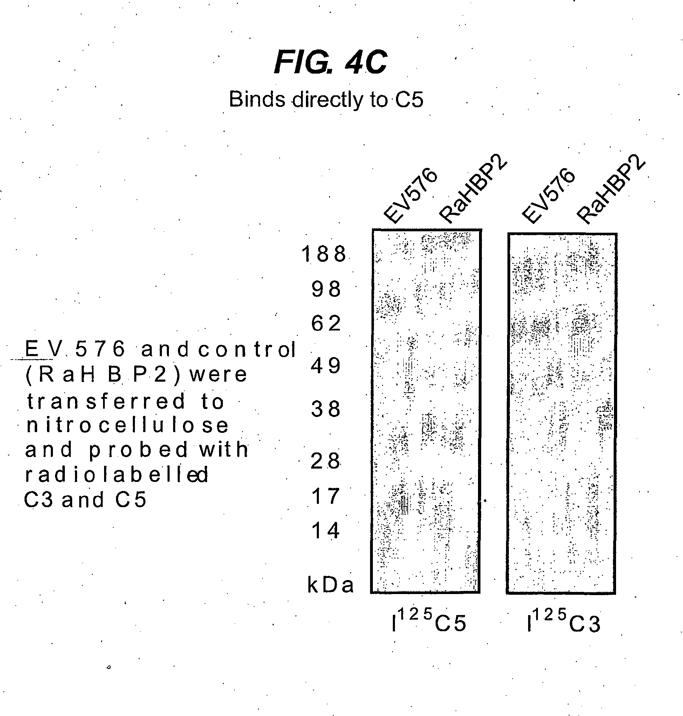

The ability of EV576 to inhibit both the classical and the alternative complement pathways is due to binding of the molecule to complement C5, the precursor of C5a and C5b - 9. EV576 binds directly to C5 (Figure 4C) with an IC₅₀ of ≈ 0.02 mg/ml. The precise binding mechanism and accessory roles (if any) played by serum factors are under investigation.

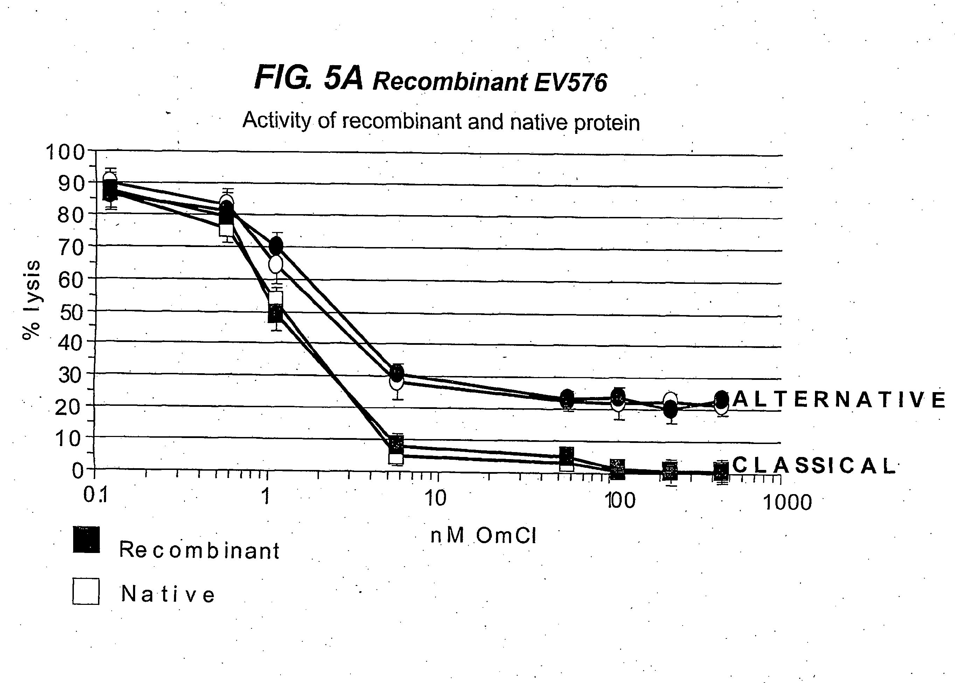

Recombinant EV576 (rEV576) with glycosylation sites removed (which otherwise are glycosylated in the yeast expression system) is as active as the native non-glycosylated protein (Figure 5A).

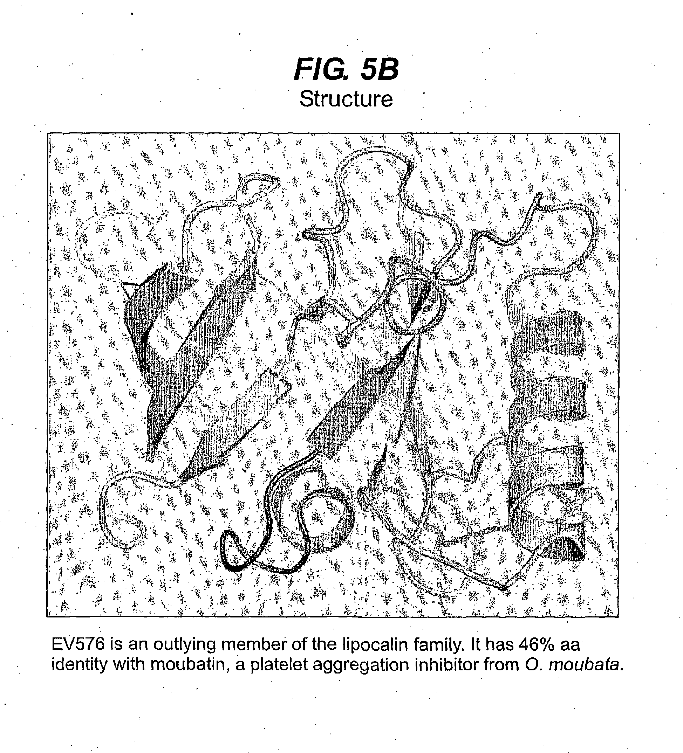

The structure of EV576 confirms that it is an outlying member of the lipocalin family (Figure 5B), having 46% identity with moubatin, a platelet aggregation inhibitor from *O*. *moubata.* Lipocalins are a large group of soft tick proteins the functions of which, with rare exceptions, are unknown.

### 2. Half-life.

The serum beta half-life of ¹²⁵I labelled rEV576 in rats was found to be ≈ 30 - 38 hours.

### 3. Effect of EV576 on experimental autoimmune myasthenia gravis.

### Experiment 1

Experimental autoimmune myasthenia gravis (EAMG) was induced in female Lewis rats according to the method of Piddlesden *et al.* (*supra*).

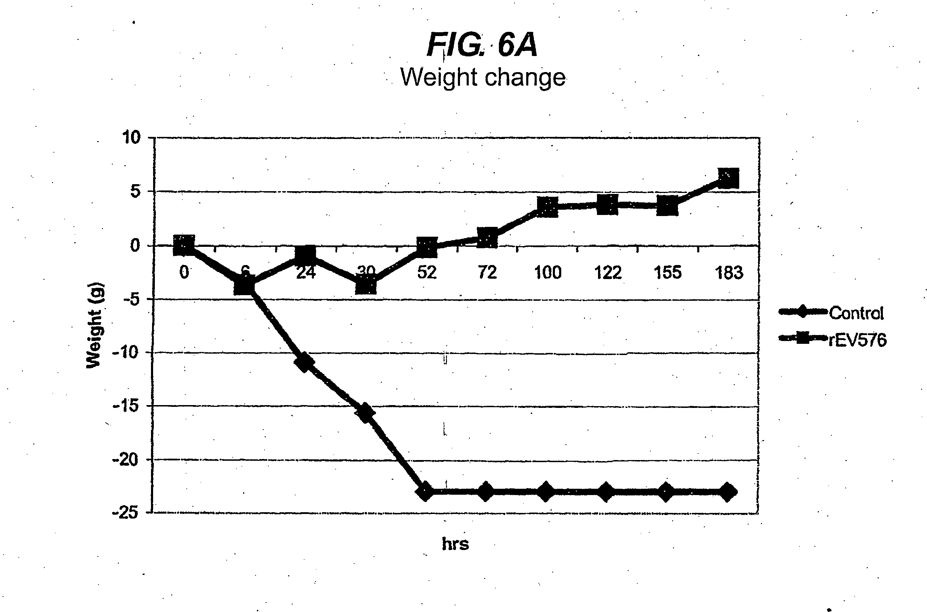

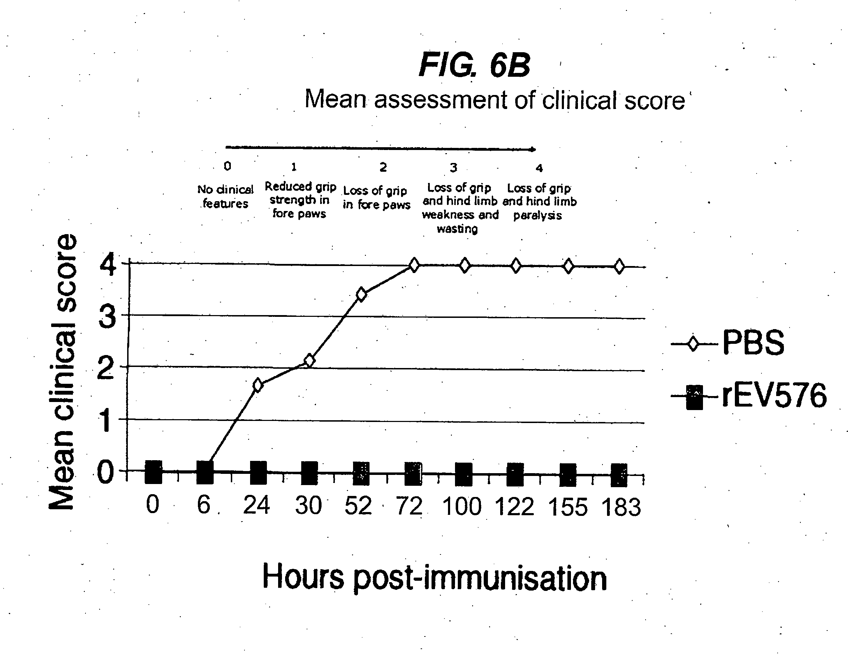

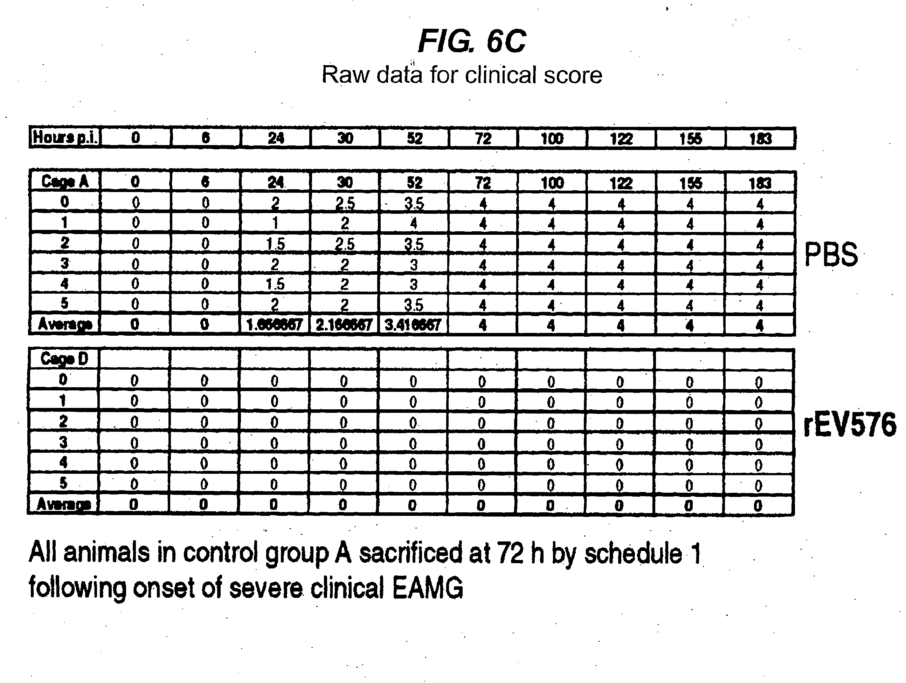

Lewis rats were injected with 1mg/kg anti-AchR mAb35 intraperitoneally at day 0, along with either: i) 3.25mg rEV576 (calculated to be 2.5 x the molar dose needed to bind all available C5); or ii) PBS. The rats were assessed for changes in weight and clinical score over 7 days. (Figure 6).

Injection of 3.25mg rEV576 totally attenuated mAb35 induced weight loss and muscular weakness compared to control.

All control animals became moribund and had to be euthanased at 72 hours post-induction whereas all rEV576 treated animals survived, gained weight and showed no muscle weakness for the duration of the experiment (183 hours) (Figure 6).

A single injection of rEV576 thus completely attenuates the symptoms of EAMG for at least 7 days.

### Experiment 2

rEV576 was further analysed in a chronic model of EAMG. In this model, animals receive acetylcholine receptor protein in Complete Freund's Adjuvant (CFA) and generate their own antibodies over the course of approximately 30 days. This model mimics the human condition where symptoms occur and progress relatively slowly.

18 Lewis rats were immunized by subcutaneous injection of purified acetylecholine receptor protein (2x20µg in 100µl of PBS emulsified in equal volume of complete Freund's adjuvant + nonviable Mycobacterium tuberculosis - 0.5mg). Control rats were injected with PBS only.

After the onset of disease (EAMG clinical score 1 or 2 and weight loss < 15%) 9 rats were injected with 3.25 mg of rEV576 i.p. and treatment continued for 10 days with 1 mg for every 12 hrs. Out of these nine rats, three rats were assessed as having severe myasthenia (S-EAMG) and six rats as having mild myasthenia (M-EAMG).

The remaining 9 rats were untreated. Out of these nine rats, four rats were assessed as having severe myasthenia (S-EAMG) and five rats as having mild myasthenia (M-EAMG).

The two sets of rats were each assessed for changes in weight, grip strength and clinical score over 10 days.

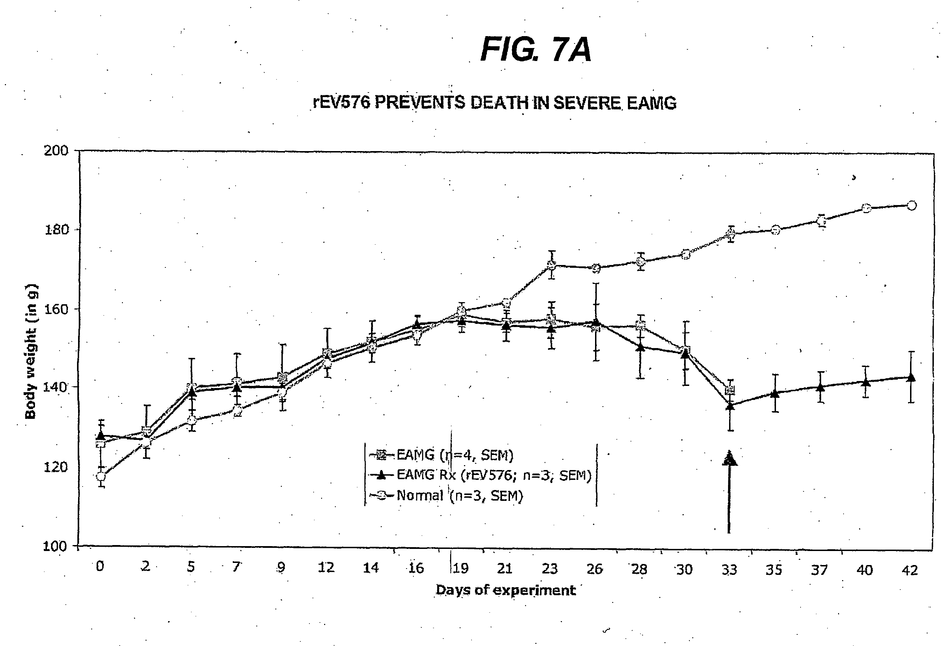

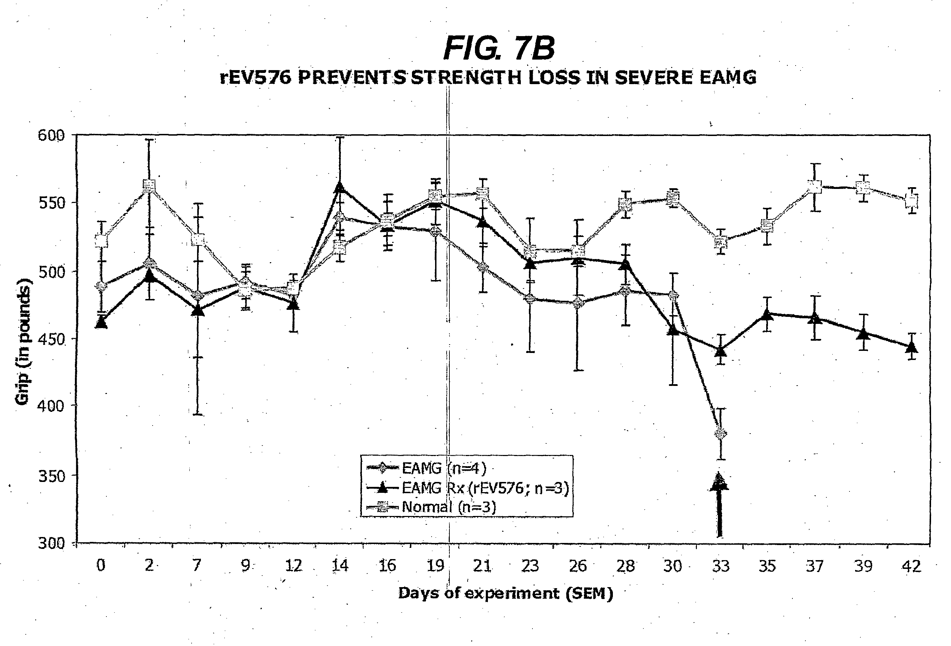

The rats in the severe EAMG group had lost 12% of their body weight and were within 24 hours of death when treatment with rEV576 was started. Untreated rats all died within 24 hours. The rEV576 treated rats showed a significant reduction in weight loss. At the beginning of treatment with rEV576 on Day 33, the average clinical score was 2.0. Injection of rEV576 for 10 days reduced the severity of clinical symptoms and prevented further weight loss (Figure 7A). These rats also showed significant improvement in grip strength (Figure 7B).

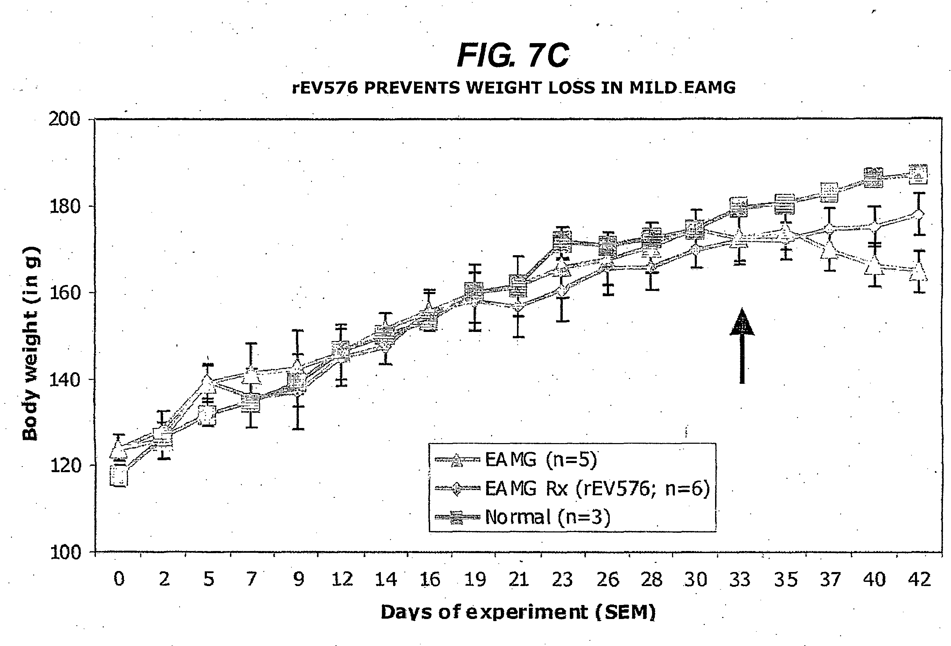

In rats exhibiting the mild early stage of EAMG, clinical signs were just starting and weight was just starting to decrease at Day 33. Weight loss was prevented in rats treated with rEV576 and rats gained in average about 3.43% of body weight. Untreated animals with identical clinical scores lost about 4.59% of body weight (Figure 7C). There was no improvement in grip strength in treated compared with untreated rats exhibiting the mild early stage of EAMG (results not shown).

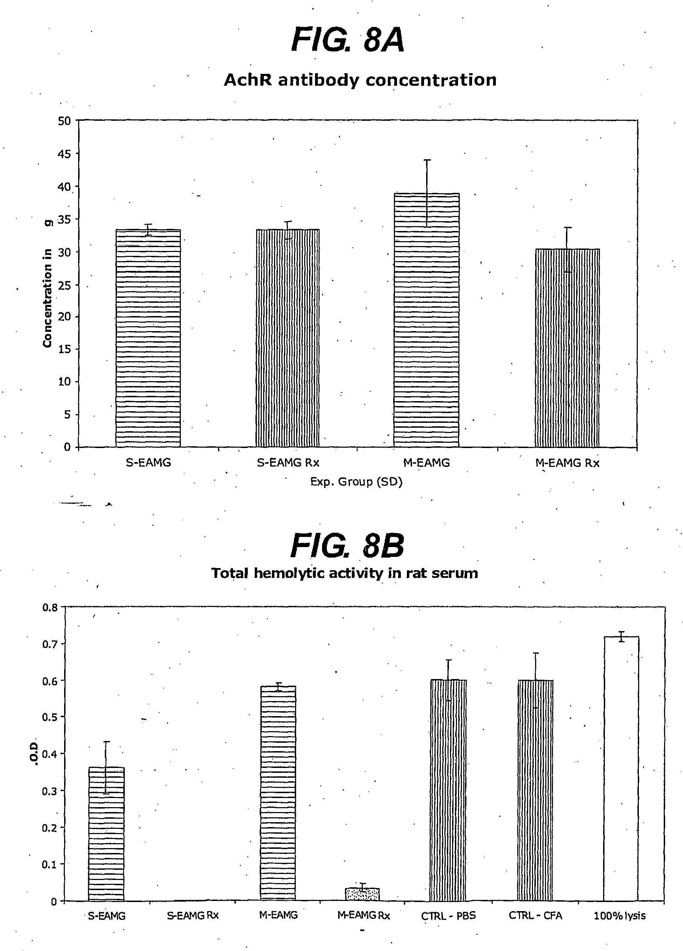

Blood was collected from the rats for the detection of AchR antibodies and an assessment of complement hemolytic activity. There was no difference between experimental groups in the AchR antibodies detected by direct ELISA (Figure 8A). Rat serum from rats exhibiting both severe and mild EAMG which were treated with rEV576 completely inhibited complement activity (Figure 8B).

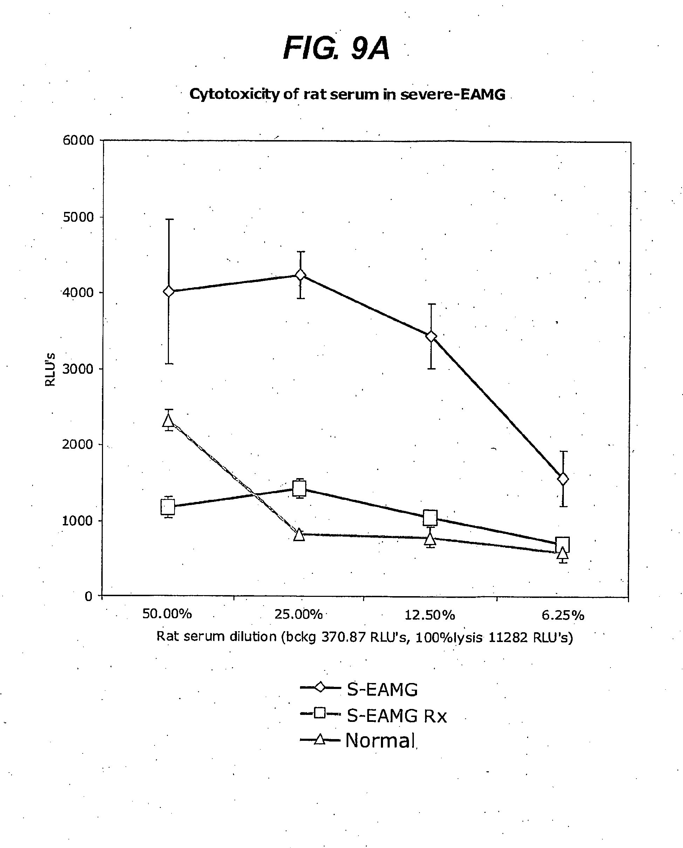

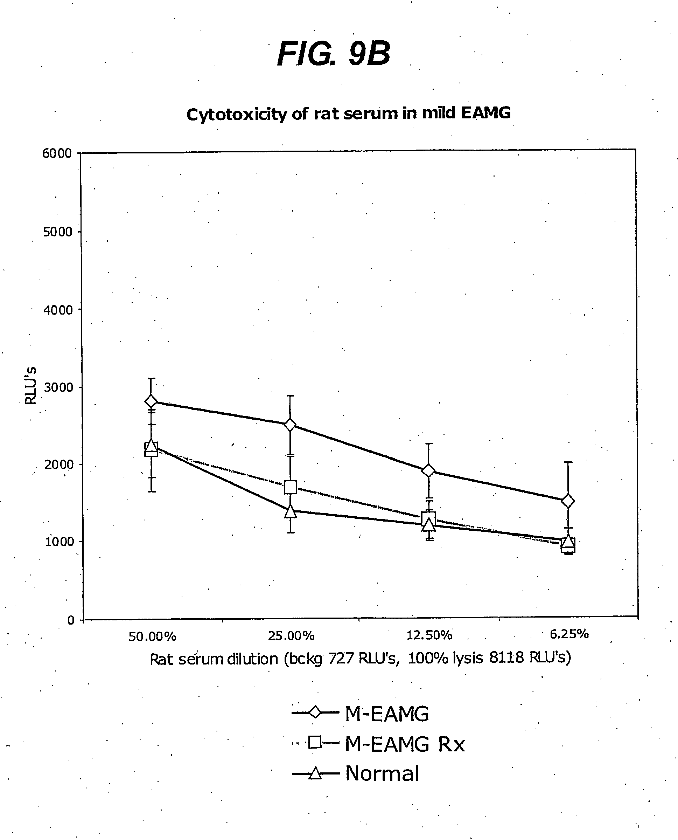

In addition, the cytotoxicity of rat serum from rats exhibiting both severe and mild EAMG was measured on rhabdomyosarcoma cell line (ATCC, CCL-136) using a ToxiLight Bioassay Kit. As can be seen in Figures 9A and 9B, the highest cytotoxicity was detected in untreated rats with severe EAMG. Animals treated with rEV576 showed a significant decrease in cytotoxic activity.

This data suggests that rEV576 may be effective in treating both early mild myasthenia gravis and severe late stage disease (eg myasthenic crises).

## Claims

1. An agent derived from a haematophagous arthropod that binds complement C5 for use in the treatment or prevention of myasthenia gravis, wherein the agent that binds complement C5 is:
(a) a protein comprising or consisting of amino acids 19 to 168 of the amino acid sequence of SEQ ID NO: 2;
(b) a protein comprising or consisting of amino acids 1 to 168 of the amino acid sequence of SEQ ID NO: 2;
(c) a homologue having greater than 60% sequence identity to the protein as defined in (a) or (b); or
(d) a fragment of the protein as defined in (a) or (b) or of a homologue as defined in (c), wherein said fragment retains the ability to bind complement C5.

2. The agent for use according to claim 1, wherein the agent acts to prevent the cleavage of complement C5 by C5 convertase into complement C5a and complement C5b-9.

3. The agent for use according to claim 1 or claim 2, wherein the agent binds C5 with an IC₅₀ of less than 0.2 mg/ml.

4. The agent for use according to any one of claims 1 to 3, wherein the agent is administered as a nucleic acid molecule encoding the protein as recited in claim 1.

5. The agent for use according to claim 4, wherein the nucleic acid molecule comprises or consists of bases 53 to 507 of the nucleotide sequence of SEQ ID NO: 1.

6. The agent for use according to claim 5, wherein the nucleic acid molecule comprises or consists of bases 1 to 507 of the nucleotide sequence of SEQ ID NO: 1.

7. The agent for use according to any one of claims 1 to 6, wherein the subject is a mammal, preferably a human.

8. The agent for use according to any one of claims 1 to 7, wherein the agent is administered in a dose sufficient to bind as much available C5 as possible in the subject, more preferably, all available C5.

9. The agent for use according to any one of claims 1 to 8, wherein the agent is administered in a dose sufficient to attenuate weight loss and/or muscle weakness.

10. The agent for use according to any one of claims 1 to 9, wherein the agent is administered in a dose from 1 mg/kg to 15 mg/kg.

11. The agent for use according to any one of claims 1 to 10, wherein the agent is administered intraperitoneally as a single dose of 13 mg/kg.

12. The agent for use according to any one of claims 1 to 9, wherein the agent is administered intraperitoneally at a dose of 13 mg/kg followed by a 12-hourly dose of 4 mg/kg.

13. The agent for use according to any one of claims 1 to 12, wherein the agent that binds C5 is administered as part of a treatment regimen also involving the administration of a further drug for the treatment of myasthenia gravis.

14. The agent for use according to claim 13, wherein the further drug is an anticholinesterase agent, such as neostigmine and pyridostigmine, or an immunosuppressive drug, such as prednisone, cyclosporine, and azathioprine.

15. The agent for use according to claim 13 or claim 14, wherein the agent that binds C5 is administered simultaneously, sequentially or separately with the further drug.

16. The agent for use according to any one of claims 1 to 15, wherein the myasthenia gravis is mild myasthenia gravis or severe myasthenia gravis.

17. Use of a therapeutically or prophylactically effective amount of an agent derived from a haematophagous arthropod that binds complement C5 in the manufacture of a medicament for treating or preventing myasthenia gravis, wherein the agent that binds complement C5 is:
(a) a protein comprising or consisting of amino acids 19 to 168 of the amino acid sequence of SEQ ID NO: 2;
(b) a protein comprising or consisting of amino acids 1 to 168 of the amino acid sequence of SEQ ID NO: 2;
(c) a homologue having greater than 60% sequence identity to the protein as defined in (a) or (b); or
(d) a fragment of the protein as defined in (a) or (b) or of a homologue as defined in (c), wherein said fragment retains the ability to bind complement C5.

## Patentansprüche

1. Von einem hämatophagen Arthropoden stammendes Agens, das Komplement C5 bindet, zur Verwendung in der Behandlung oder Vorbeugung von Myasthenia gravis, wobei es sich bei dem Agens, das Komplement C5 bindet, um Folgendes handelt:
(a) ein Protein, umfassend die bzw. bestehend aus den Aminosäuren 19 bis 168 der Aminosäuresequenz gemäß SEQ ID NO: 2;
(b) ein Protein, umfassend die bzw. bestehend aus den Aminosäuren 1 bis 168 der Aminosäuresequenz gemäß SEQ ID NO: 2;
(c) ein Homolog mit mehr als 60% Sequenzidentität zu dem in (a) oder (b) definierten Protein; oder
(d) ein Fragment des in (a) oder (b) definierten Proteins oder eines wie in (c) definierten Homologs, wobei das Fragment die Fähigkeit, Komplement C5 zu binden, beibehält.

2. Agens zur Verwendung nach Anspruch 1, wobei das Agens dahingehend wirkt, dass es die Spaltung von Komplement C5 durch die C5-Konvertase in Komplement C5a und Komplement C5b-9 verhindert.

3. Agens zur Verwendung nach Anspruch 1 oder Anspruch 2, wobei das Agens C5 mit einem IC₅₀-Wert von weniger als 0,2 mg/ml bindet.

4. Agens zur Verwendung nach einem der Ansprüche 1 bis 3, wobei das Agens als Nukleinsäuremolekül, das für das Protein wie in Anspruch 1 genannt codiert, verabreicht wird.

5. Agens zur Verwendung nach Anspruch 4, wobei das Nukleinsäuremolekül die Basen 53 bis 507 der Nukleotidsequenz gemäß SEQ ID NO: 1 umfasst oder aus diesen besteht.

6. Agens zur Verwendung nach Anspruch 5, wobei das Nukleinsäuremolekül die Basen 1 bis 507 der Nukleotidsequenz gemäß SEQ ID NO: 1 umfasst oder aus diesen besteht.

7. Agens zur Verwendung nach einem der Ansprüche 1 bis 6, wobei es sich bei dem Individuum um ein Säugetier, vorzugsweise einen Menschen, handelt.

8. Agens zur Verwendung nach einem der Ansprüche 1 bis 7, wobei das Agens in einer Dosis verabreicht wird, die ausreicht, um so viel verfügbares C5 wie möglich, stärker bevorzugt alles verfügbare C5, in dem Individuum zu binden.

9. Agens zur Verwendung nach einem der Ansprüche 1 bis 8, wobei das Agens in einer Dosis verabreicht wird, die ausreicht, um Gewichtsverlust und/oder Muskelschwäche zu lindern.

10. Agens zur Verwendung nach einem der Ansprüche 1 bis 9, wobei das Agens in einer Dosis von 1 mg/kg bis 15 mg/kg verabreicht wird.

11. Agens zur Verwendung nach einem der Ansprüche 1 bis 10, wobei das Agens intraperitoneal als Einzeldosis von 13 mg/kg verabreicht wird.

12. Agens zur Verwendung nach einem der Ansprüche 1 bis 9, wobei das Agens intraperitoneal bei einer Dosis von 13 mg/kg und anschließend durch eine Dosis von 4 mg/kg alle 12 Stunden verabreicht wird.

13. Agens zur Verwendung nach einem der Ansprüche 1 bis 12, wobei das Agens, das C5 bindet, als Teil eines Behandlungsschemas verabreicht wird, das auch die Verabreichung eines weiteren Arzneistoffs für die Behandlung von Myasthenia gravis beinhaltet.

14. Agens zur Verwendung nach Anspruch 13, wobei es sich bei dem weiteren Arzneistoff um ein Anticholinesterase-Agens wie Neostigmin und Pyridostigmin oder um einen immunsupprimierenden Arzneistoff wie Prednison, Cyclosporin und Azathioprin handelt.

15. Agens zur Verwendung nach Anspruch 13 oder Anspruch 14, wobei das Agens, das C5 bindet, gleichzeitig mit, nacheinander mit oder getrennt von dem weiteren Arzneistoff verabreicht wird.

16. Agens zur Verwendung nach einem der Ansprüche 1 bis 15, wobei es sich bei der Myasthenia gravis um milde Myasthenia gravis oder schwere Myasthenia gravis handelt.

17. Verwendung einer therapeutisch oder prophylaktisch wirksamen Menge eines von einem hämatophagen Arthropoden stammenden Agens, das Komplement C5 bindet, bei der Herstellung eines Medikaments zur Verwendung in der Behandlung oder Vorbeugung von Myasthenia gravis, wobei es sich bei dem Agens, das Komplement C5 bindet, um Folgendes handelt:
(a) ein Protein, umfassend die bzw. bestehend aus den Aminosäuren 19 bis 168 der Aminosäuresequenz gemäß SEQ ID NO: 2;
(b) ein Protein, umfassend die bzw. bestehend aus den Aminosäuren 1 bis 168 der Aminosäuresequenz gemäß SEQ ID NO: 2;
(c) ein Homolog mit mehr als 60% Sequenzidentität zu dem in (a) oder (b) definierten Protein; oder
(d) ein Fragment des in (a) oder (b) definierten Proteins oder eines wie in (c) definierten Homologs, wobei das Fragment die Fähigkeit, Komplement C5 zu binden, beibehält.

## Revendications

1. Agent dérivé d'un arthropode hématophage qui se lie au complément C5 pour utilisation dans le traitement prophylactique ou thérapeutique de la myasthénie grave, où l'agent qui se lie au complément C5 est :
(a) une protéine comprenant ou constituée des acides aminés 19 à 168 de la séquence d'acides aminés SEQ ID NO: 2;
(b) une protéine comprenant ou constituée des acides aminés 1 à 168 de la séquence d'acides aminés SEQ ID NO: 2;
(c) un homologue présentant une identité de séquence supérieure à 60 % à la protéine comme défini dans (a) ou (b) ; ou
(d) un fragment de la protéine comme défini dans (a) ou (b) ou d'un homologue comme défini dans (c), où ledit fragment conserve la capacité à se lier au complément C5.

2. Agent pour utilisation selon la revendication 1, où l'agent agit pour prévenir le clivage du complément C5 par la C5 convertase en le complément C5a et le complément C5b-9.

3. Agent pour utilisation selon la revendication 1 ou la revendication 2, où l'agent se lie à C5 avec une CI₅₀ de moins de 0,2 mg/ml.

4. Agent pour utilisation selon l'une quelconque des revendications 1 à 3, où l'agent est administré sous forme d'une molécule d'acide nucléique codant la protéine selon la revendication 1.

5. Agent pour utilisation selon la revendication 4, où la molécule d'acide nucléique comprend ou est constituée des bases 53 à 507 de la séquence nucléotidique SEQ ID NO: 1.

6. Agent pour utilisation selon la revendication 5, où la molécule d'acide nucléique comprend ou est constituée des bases 1 à 507 de la séquence nucléotidique SEQ ID NO: 1.

7. Agent pour utilisation selon l'une quelconque des revendications 1 à 6, où le sujet est un mammifère, préférentiellement un humain.

8. Agent pour utilisation selon l'une quelconque des revendications 1 à 7, où l'agent est administré à une dose suffisante pour se lier à autant de C5 disponible que possible chez le sujet, plus préférentiellement tout le C5 disponible.

9. Agent pour utilisation selon l'une quelconque des revendications 1 à 8, où l'agent est administré à une dose suffisante pour atténuer la perte de poids et/ou la faiblesse musculaire.

10. Agent pour utilisation selon l'une quelconque des revendications 1 à 9, où l'agent est administré à une dose comprise entre 1 mg/kg et 15 mg/kg.

11. Agent pour utilisation selon l'une quelconque des revendications 1 à 10, où l'agent est administré par voie intrapéritonéale sous forme d'une dose unique de 13 mg/kg.

12. Agent pour utilisation selon l'une quelconque des revendications 1 à 9, où l'agent est administré par voie intrapéritonéale à une dose de 13 mg/kg suivie d'une dose à 12 heures de 4 mg/kg.

13. Agent pour utilisation selon l'une quelconque des revendications 1 à 12, où l'agent qui se lie à C5 est administré dans le cadre d'un régime posologique impliquant également l'administration d'un médicament supplémentaire destiné au traitement de la myasthénie grave.

14. Agent pour utilisation selon la revendication 13, où le médicament supplémentaire est un agent de type anticholinestérase, tel que la néostigmine et la pyridostigmine, ou un médicament immunosuppresseur, tel que la prednisone, la cyclosporine et l'azathioprine.

15. Agent pour utilisation selon la revendication 13 ou la revendication 14, où l'agent qui se lie à C5 est administré simultanément, séquentiellement ou séparément vis-à-vis du médicament supplémentaire.

16. Agent pour utilisation selon l'une quelconque des revendications 1 à 15, où la myasthénie grave est la myasthénie grave légère ou la myasthénie grave sévère.

17. Utilisation d'une quantité thérapeutiquement ou prophylactiquement active d'un agent dérivé d'un arthropode hématophage qui se lie au complément C5 dans la fabrication d'un médicament destiné au traitement prophylactique ou thérapeutique de la myasthénie grave, où l'agent qui se lie au complément C5 est :
(a) une protéine comprenant ou constituée des acides aminés 19 à 168 de la séquence d'acides aminés SEQ ID NO: 2;
(b) une protéine comprenant ou constituée des acides aminés 1 à 168 de la séquence d'acides aminés SEQ ID NO: 2;
(c) un homologue présentant une identité de séquence supérieure à 60 % à la protéine comme défini dans (a) ou (b) ; ou
(d) un fragment de la protéine comme défini dans (a) ou (b) ou d'un homologue comme défini dans (c), où ledit fragment conserve la capacité à se lier au complément C5.


### SEQUENCE LISTING

<110> EVOLUTEC LIMITED
<120> METHOD OF TREATING MYASTHENIA GRAVIS
<130> P041905WO
<150> 0518443.7 <151> 2005-09-09
<160> 2
<170> SeqWin99, version 1.02
<210> 1
   <211> 507
   <212> DNA
   <213> Orthinodoros moubata
<400> 1
   atgctggttt tggtgaccct gattttctcc ttttctgcga acatcgcata tgctgacagc   60
   gaaagcgact gcactggaag cgaacctgtt gacgccttcc aagctttcag tgagggcaaa  120
   gaggcatatg tcctggtgag gtccacggat cccaaagcga gggactgctt gaaaggagaa  180
   ccagccggag aaaagcagga caacacgttg ccggtgatga tgacgtttaa gaatggcaca  240
   gactgggctt caaccgattg gacgtttact ttggacggcg caaaggtaac ggcaaccctt  300
   ggtaacctaa cccaaaatag ggaagtggtc tacgactcgc aaagtcatca ctgccacgtt  360
   gacaaggtcg agaaggaagt tccagattat gagatgtgga tgctcgatgc gggagggctt  420
   gaagtggaag tcgagtgctg ccgtcaaaag cttgaagagt tggcgtctgg caggaaccaa  480
   atgtatcccc atctcaagga ctgctag                                      507
<210> 2
   <211> 168
   <212> PRT
   <213> Orthinodoros moubata
<400> 2
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Asn Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Asn Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165